# EUROPÄISCHE PATENTSCHRIFT

(11) **EP 0 626 145 B1**
(45) Veröffentlichungstag und Bekanntmachung des Hinweises auf die Patenterteilung: **09.07.1997**
(21) Anmeldenummer: 94105260.7
(22) Anmeldetag: 05.04.1994
(51) Int. Cl.: A47B 88/12, A61L 2/26

(54) **Anordnung zur Lagerung von Sterilgut-Containern oder dergleichen**
Device for stockage of sterile containers or similair
Dispositif de stockage pour des récipients stériles, ou similaires

(30) Priorität: 21.05.1993 DE 4317138
(43) Veröffentlichungstag der Anmeldung: 30.11.1994
(73) Patentinhaber: METALLWARENFABRIK WALTER H. BECKER GmbH, 84371 Triftern (DE)
(72) Erfinder: Becker, Hans Werner, D-84371 Triftern (DE)
(74) Vertreter: Reinhard - Skuhra - Weise & Partner

(56) Entgegenhaltungen:
- DE-A- 3 534 838
- DE-A- 4 226 951
- FR-A- 2 676 342
- GB-A- 2 144 029
- US-A- 4 199 200

## Beschreibung

Die Erfindung betrifft eine Anordnung zur Lagerung von Sterilgut-Containern oder dergleichen gemäß dem Oberbegriff des Patentanspruchs 1.

Bekannte Anordnungen zur Lagerung von Sterilgut-Containern innerhalb von Regalen, Schränken oder dergleichen, insbesondere in Krankenhäusern, bestehen aus in Horizontalrichtung verlaufenden, im wesentlichen U-förmigen Führungsschienen, in welche Drahtkörbe eingesetzt sind, auf welche Container oder andere Behältnisse aufgesetzt werden können, um in das Regal, den Schrank oder dergleichen eingeschoben zu werden. Zur definierten Herausverlagerung dieser Körbe sind diese durch Arretierstifte oder dergleichen gegenüber den Führungsschienen gesichert.

Nachteilig bei der bekannten Anordnung ist der Einsatz von im wesentlichen U-förmigen Führungsschienen, die schwer zugänglich sind und mangels ausreichender Reinigungsmöglichkeit die erforderlichen Hygienebedingungen nicht erfüllen. U-förmige oder winkelförmige Führungsschienen sind nicht nur wegen Ihrer schlechten Zugänglichkeit äußerst schwierig zu reinigen, sie weisen auch eine vergleichbar große Fläche auf, die somit einer starken Kontaminierung unterliegt.

Der Erfindung liegt daher die Aufgabe zugrunde, eine Anordnung gemäß dem Oberbegriff des Patentanspruchs 1 so auszubilden, daß sie hygienefreundlich ist und auf einfache und sichere Weise benutzt werden kann.

Diese Aufgabe wird erfindungsgemäß durch die im Patentanspruch 1 angegebenen Merkmale gelöst.

Weitere Ausgestaltungen der erfindungsgemäßen Anordnung ergeben sich aus den Unteransprüchen.

Die Erfindung schafft eine Anordnung, insbesondere zur Lagerung von Sterilgut-Containern in Krankenhäusern, die eine Führungseinrichtung mit vergleichbar geringer Fläche aufweist und einen durch die Führungseinrichtung geführten Korb, der ohne Gefahr eines Verkippens in beiden Richtungen in die Führungseinrichtung einsetzbar ist. Dabei braucht nicht auf eine bestimmte Einschubrichtung des Korbes geachtet werden.

Gemäß einer bevorzugten Ausgestaltung der Erfindung sind als Führungsnuten seitlich am Korb angebrachte Bügel vorgesehen, die eine Länge haben, die etwa einem Viertel der Länge des Korbes entspricht und auf diese Weise die Auszugstiefe auf ein Viertel der Korblänge festlegen.

Gemäß einer bevorzugten Ausführungsform der Erfindung sind die Führungsnuten zur Mitte des Korbes hin geöffnet und zu den Stirnseiten des Korbes hin geschlossen, wodurch ein unbeabsichtigtes Entfernen des Korbes und/oder ein Herauskippen des Korbes aus dem zugehörigen Regal, Schrank oder dergleichen wirksam verhindert wird.

Gemäß einer weiteren Ausgestaltung der Erfindung werden Führungbolzen verwendet, die profiliert sind bzw. eine Nut oder eine umlaufende Nut aufweisen. Die innerhalb der Nut laufenden Stäbe des Korbes liegen damit im wesentlichen nur punktförmig oder entlang eines Teilkreises auf den Führungsbolzen auf, infolgedessen auch ein Durchbiegen des Korbes selbst bei aufgesetzten Containern oder dergleichen sowie ein Abgleiten der Führungsstäbe des Korbes aus beziehungweise von dem Führungsbolzen verhindert wird.

Der auf dem Bolzen geführte Führungsstab dient gleichzeitig als seitliche Begrenzung des Korbes und ist an den Enden des Korbes im wesentlichen bzw. vorzugsweise vertikal nach unten gebogen, wodurch die Führungsnut gegenüber den Stirnkanten des Korbes begrenzt ist.

Gemäß einer bevorzugten Ausführungsform der Erfindung weist der Korb an beiden Seiten einen hochgezogenen Bügel auf, in welchen der Führungsbolzen zur Vermeidung eines Kippens eingreift und dadurch eine horizontale Führung des Korbes bei Belastung sicherstellt.

Die erfindungsgemäße Anordnung hat den Vorteil, daß die Führungsbolzen durch Wisch- oder Sprühdesinfektion jederzeit und einfach zu reinigen sind bzw. desinfiziert werden können. Der Korb selbst läßt sich mit handelsüblilchen Desinfektionsmaschinen desinfizieren oder in handelsüblichen Sterilisatoren sterilisieren.

Die Befestigung der Führungsbolzen erfolgt vorzugsweise durch Verschweißen am entsprechenden Rahmen bzw. Schrank oder Regal, infolgedessen keine zusätzlichen Öffnungen wie Gewindeöffnungen usw. in Hohlkörpern erforderlich sind.

Die erfindungsgemäße Anordnung läßt sich in herkömmlichen Schränken, Regalen oder auch Transportwagen, wie sie insbesondere vom Krankenhausbedarf her bekannt sind, einsetzen.

Im folgenden werden bevorzugte Ausführungsformen der erfindungsgemäßen Anordnung zur Erläuterung weiterer Merkmale und Vorteile beschrieben.

Es zeigen:
- Fig. 1: eine schematische Draufsicht auf eine bevorzugte Ausführungsform der Anordnung,
- Fig. 2: die Ansicht einer Seite des Korbes mit Führungsbolzen,
- Fig. 3: eine Ansicht der anderen Seite des Korbes, und
- Fig. 4: eine schematische Darstellung eines Führungsbolzens, im Schnitt.

Nachfolgend werden bevorzugte Ausführungsformen der erfindungsgemäßen Anordnung beschrieben.

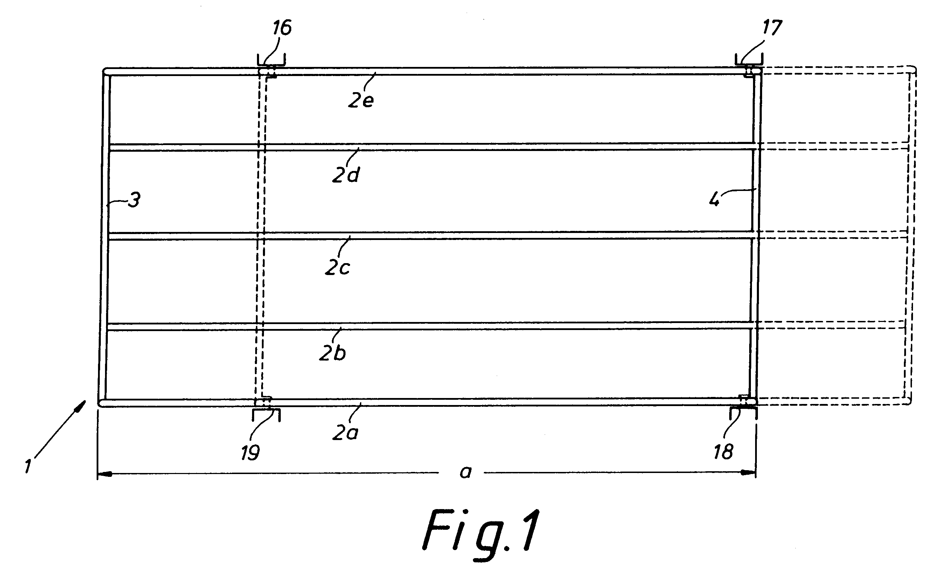

Fig. 1 bis 3 zeigen Einzelheiten des bei der erfindungsgemäßen Anordnung verwendeten Korbes 1. Ein derartiger Korb 1 dient als Auflage für Container oder andere Gegenstände und wird durch parallele Gitterstäbe 2a, 2b usw. gebildet, die eine ebene Auflagefläche festlegen. Vorzugsweise bestehen die Gitterstäbe 2a, 2b usw. wie auch alle Teile dieses Korbes aus Chromnickelstahl.

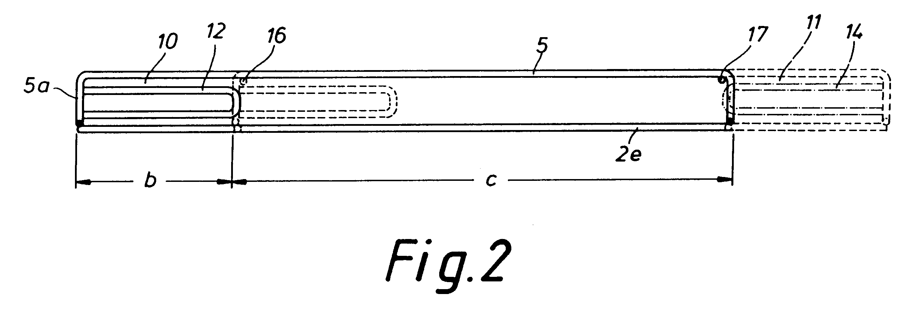

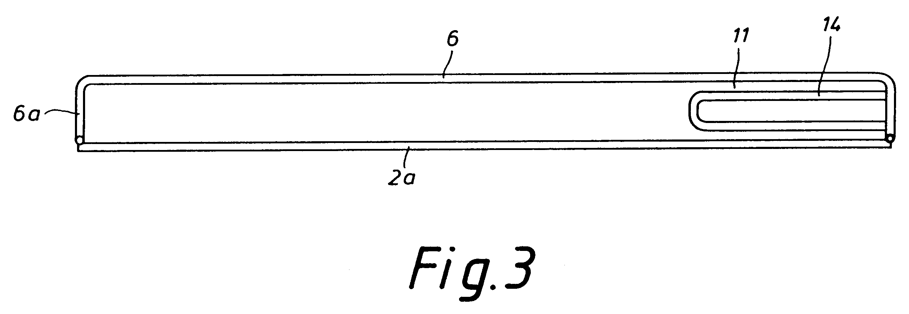

Gemäß Fig. 1 sind die Gitterstäbe 2a, 2b usw. an einem Querstab 3, 4 an den Stirnseiten des Korbes fixiert, beispielsweise durch Verschweißung. Seitlich des in Fig. 1 gezeigten Korbes 1 sind entsprechend Fig. 2 und Fig. 3 weitere, hochgezogene Stäbe 5, 6 vorgesehen, die eine seitliche Begrenzung des Korbes 1 ergeben, oder als seitliche Führung für auf den Korb 1 abgestellte Gegenstände dienen.

Der erfindungsgemäße Korb 1 weist durch die Gitterstäbe 5, 6 eine seitliche Begrenzung auf, die eine Fläche definiert, die im wesentlichen vertikal steht zum durch die Gitterstäbe 2a, 2b usw. 3, 4 definierten Korbboden. Im Bereich dieser Seitenbegrenzungen sind parallel zum Korbboden verlaufende Führungsnuten 10, 11 vorgesehen. Die Führungsnuten 10, 11 werden durch einen Bogen 12, 14 in einer zum Boden des Korbes 1 parallelen Richtung festgelegt. Jeder Bogen 11, 12 ist an der vorderen Stirnseite des Korbes 1 am zugehörigen Gitterstab 5, 6 befestigt, vorzugsweise an einem vertikal verlaufenden Bereich 5a bzw. 6a des Gitterstabes 5, 6. Auf diese Weise ist die Führungsnut 10 bzw. 11 jeweils in einer Richtung geöffnet, die zum Befestigungsbereich des Bogens 12, 14 abgewandt ist. Somit öffnet sich jede Führungsnut 10, 11 in Richtung auf die Mitte des Korbes 1, wie aus den Fig. 2 und 3 ersichtlich ist. Jede Nut 10, 11 verläuft in einem Abstand zu dem durch die Gitterstäbe 2a, 2b usw. definierten Korbboden und hat eine Breite, die derart gewählt ist, daß ein Führungsbolzen 16, 17 usw. innerhalb der Führungsnuten 10, 11 verlagerbar sind.

Bei der in den Fig. 1 bis 3 gezeigten Ausführungsformen sind die Führungsnuten 10, 11 in Bezug auf den Korb 1 so vorgesehen, daß die Führungsnut 10 am vorderen rechten Ende und die Führungsnut 11 am hinteren linken Ende des Korbes 1 zu liegen kommt. Sobald der Korb 1 in das zugehörige Schrank- oder Regalfach eingesetzt ist, befinden sich zwei der Führungsbolzen 16, 17 usw. in Eingriff mit der Führungsnut bzw. 11, das heißt diagonal einander gegenüberliegende Führungsnuten 10, 11 befinden sich jeweils in Eingriff mit den Führungsbolzen 10 bzw. 18, was zur Folge hat, daß der Korb 1 auf diagonal gegenüberliegenden Seiten durch die Führungsbolzen 16 bis 19 geführt ist und dadurch eine Sicherheit gegen Verkippen des Korbes 1 gewährleistet ist.

Gemäß einer abgewandelten Ausführungsform des Korbes 1 sind an allen vier Ecken derartige Führungsnuten 10, 11 ausgebildet, was jedoch zu einem größeren Aufwand an Material und Herstellungskosten führt.

Die Führungsbolzen 16 bis 19 sind in dem den Korb 1 aufnehmenden Schrank in einer horizontalen Ebene unter Einhaltung eines Abstandes c vorgesehen, der gleich der Differenz aus a - b ist, wobei b die Länge der Bügel 12, 14 bzw. der Nuten 10, 11 darstellt, und a die Gesamtlänge des Korbes 1 ist. Dabei entspricht b etwa einem Viertel der Gesamtlänge a. Gemäß der Erfindung läßt sich der Korb 1 um ein Viertel seiner Länge entsprechend der Dimensionierung b aus dem Schrank, Regal oder dergleichen herausziehen, worauf hin dann ein Container oder ein anderes Element aufgesetzt und zusammen mit dem Korb 1 wieder in den Schrank hingeschoben werden kann. Ein Verkippen des Korbes 1 läßt sich sowohl bei Anwendung von nur zwei diagonal einander gegenüberliegenden Führungsnuten 10, 11 oder auch bei vier, an allen Ecken des Korbes 1 ausgebildeten Führungsnuten sicher vermeiden.

Die in Verbindung mit Fig. 1 bis 3 beschriebene Anordnung läßt sich mehrfach und übereinander in einem Schrank, Regal, Transportwagen oder dergleichen vorsehen, so daß eine Mehrzahl von Einschüben realisierbar ist.

Die Führungsbolzen 16, 17, 18, 19 sind in dem betreffenden Schrank oder dergleichen an zugehörigen Rahmenteilen oder Edelstahlrohren befestigt. Vorzugsweise werden die Führungsbolzen durch ein Bolzenschweißverfahren am Rahmen oder dergleichen befestigt, was das Einbringen von Gewindebohrungen in die Rahmenteile erübrigt.

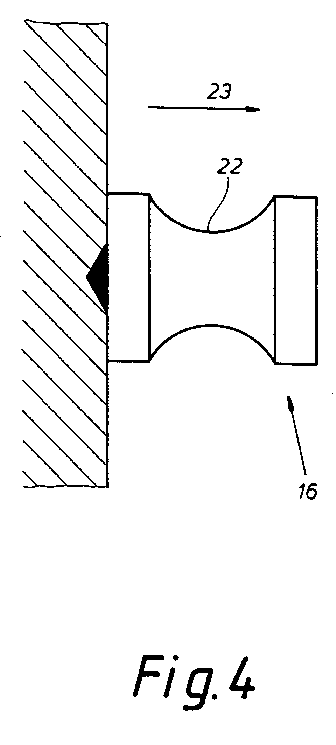

Die Führungsbolzen 16 bis 18 haben vorzugsweise eine umlaufende Nut 22. Ein Beispiel eines Führungsbolzens ist in Fig. 4 im Schnitt dargestellt. Die Nut 22 des in Fig. 4 gezeigten, mit 16 bezeichneten Führungsbolzens hat kreisförmigen oder teilkreisförmigen Querschnitt, wobei der Radius der Nutwand vorzugsweise dem Radius der verwendeten Gitterstäbe 5,6 entspricht, die entlang und auf den Führungsbolzen 16 bis 19 gleiten. Dadurch bilden die Nuten 22 eine Aufnahme für die jeweils zueinander parallelen Gitterstäbe 5, 6 und verhindern ein Durchbiegen des Korbes 1 im Belastungsfalle, da die Gitterstäbe 5, 6 durch die Formgebung der Nuten 22 an einer Verlagerung aus den Nuten 22 heraus gehindert werden. Bei zylindrischen Führungsbolzen 16 bis 28 ist dagegen eine Verlagerung der Gitterstäbe 5, 6 im Belastungsfalle in Richtung des in Fig. 4 gezeigten Pfeiles 23 möglich.

Die Breite der Führungsnuten 10, 11 ist bei einer ersten Ausführungsform geringfügig größer als der Außendurchmesser der Führungsbolzen 16 usw. gewählt. Gegebenenfalls kann jede Nut 10, 11 auch von solcher Breite sein, daß der Führungsbolzen mit einer umlaufenden Nut 22 sowohl an dem oberen Gitterstab 5 bzw. als auch an dem unteren, den Bogen 11 bzw. 12 definierenden, gebogenen Gitterstab in Anlage gelangt.

Bei der erfindungsgemäßen Anordnung wird folgendes gewährleistet: in jeder Stellung des Korbes 1 mit Ausnahme der "vollständig eingefahrenen Stellung" befindet sich jeweils ein rückseitig als auch ein vorderseitig angeordneter Führungsbolzen in Eingriff mit der jeweils zugeordneten Führungsnut 10 bzw. 11. Aus Fig. 1 udn 2 ist ersichtlich, daß der Korb zwischen zwei Positionen verstellbar ist, nämlich zwischen der ausgefahrenen Position, in welcher der Korb in Fig. 1 und 2 voll ausgezeichnet dargestellt ist, und der eingefahrenen Position, die durch die strichlierte Darstellung gezeigt ist. Wie sich weiter aus Fig. 2 ergibt, befindet sich in der eingefahrenen Position des Korbes, die der strichlierten Stellung entspricht, der in Fig. 2 rechte Bolzen 17 außerhalb der "hinteren" Führungsnut 11. In dieser Position läßt sich der Korb gegenüber jedem hinteren Führungsbolzen 17 aushängen, indem die hintere Kante des Korbes angehoben wird. Die darauf folgende Verlagerung des Korbes in Fig. 2 nach links führt dazu, daß der Korb wegen des in außer Eingriff befindlichen Zustandes zwischen dem Bolzen 17 und der Nut 11 über die in Fig. 2 dargestellte ausgefahrene Position nach links herausgefahren werden kann und auf diese Weise auch aus dem zugehörigen Rahmen verlagerbar ist.

Wie sich aus vorstehender Beschreibung ergibt, ist der Abstand zwischen den Bolzen 16 und 17 kleiner als der Abstand der Beginn der einander zugeordneten Nuten 10 und 11, wodurch in der vorstehend beschriebenen Weise das Aus- und Einhängen des Korben bewerkstelligt werden kann.

Der beschriebene Korb 1 ermöglicht einen durch die Anordnung der Bolzen 16 bis 19 einerseits und die Länge der Bügel 11 bzw. 12 andererseits festgelegte Auszugslänge. Jeder Korb 1 läßt sich auf einfache Weise aus seiner Führungsposition aus dem Schrank, Regal oder dergleichen entfernen und auf einfache Weise wieder einführen. Weiterhin lassen sich die Führungsbolzen 16 bis 19 mit geringem Aufwand und auf einfache und sichere Weise reinigen.

Die Führungsbolzen 16 bis 19 bestehen ebenso wie die einzelnen Stäbe des Korbes 1 vorzugsweise aus Chromnickelstahl.

Gemäß einer weiteren Abwandlung der Erfindung kann der Boden des Korbes 1 oberhalb der Führungsnuten 10 bzw. 11, das heißt oberhalb der die Führungsnuten festlegenden Bögen 12 bzw. 14 festgelegt sein. Die Form des Korbes 1 kann entsprechend den Bedürfnissen geändert werden, ohne daß die vorstehend beschriebene Funktionsweise dadurch verändert wird.

Aus vorstehender Beschreibung ist ersichtlich, daß die Breite des Korbes 1 den jeweiligen Bedürfnissen entsprechend angepaßt werden kann, ebenso die Länge des jeweiligen Korbes. Der Begriff "Korb" bedeutet hier grundsätzlich ein Aufnahmeelement oder eine Drahtablage, die gemäß einer bevorzugten Ausführungsform aus einzelnen, zueinander beabstandeten Drähten oder Drahtstäben zusammengesetzt ist.

## Patentansprüche

1. Anordnung zur Lagerung von Sterilgut-Containern oder dergleichen,
mit einer in einen Schrank, ein Regal oder dergleichen eingesetzten Führungseinrichtung zur Führung eines Korbes (1),
**dadurch gekennzeichnet**,
daß die Führungseinrichtung aus Führungsbolzen (16 bis 19) besteht, die eine Führungsebene festlegen,
daß der Korb (1) parallel zur Führungsebene verlaufende Führungsnuten (10, 11) aufweist,
daß die Führungsnuten (10, 11) zumindest diagonal zueinander im Bereich der Ecken des Korbes (1) ausgebildet sind,
daß die Führungsbolzen (16 bis 19) in Bewegungsrichtung des Korbes (1) einen Abstand einhalten, der etwa um die Länge der Führungsnut (10, 11) kleiner ist als die Länge (a) des Korbes (1).

2. Anordnung nach Anspruch 1,
**dadurch gekennzeichnet**,
daß jede Führungsnut (10, 11) in Richtung der Mitte des Korbes (1) geöffnet ist.

3. Anordnung nach Anspruch 1 oder 2,
**dadurch gekennzeichnet**,
daß jede Führungsnut (10, 11) durch ein seitlich am Korb (1) verlaufenden Bügel (12, 14) oder dergleichen festgelegt ist.

4. Anordnung nach wenigstens einem der vorangehenden Ansprüche,
**dadurch gekennzeichnet**,
daß die Führungsbolzen (16 bis 19) mit einer Führungsnut (22) versehen sind.

5. Anordnung nach Anspruch 4,
**dadurch gekennzeichnet**,
daß die Führungsbolzen (16 bis 19) jeweils eine umlaufende Führungsnut (22) aufweisen.

6. Anorndung nach wenigstens einem vorangehenden Ansprüche,
**dadurch gekennzeichnet**,
daß die Führungsbolzen (16 bis 19) an einem Rahmen oder dergleichen angeschweißt sind.

7. Anordnung nach Anspruch 3,
**dadurch gekennzeichnet**,
daß die Bügel (12, 14) seitlich des Korbes (1) vorgesehen sind.

8. Anordnung nach wenigstens einem der vorangehenden Ansprüche,
**dadurch gekennzeichnet**,
daß die Krümmung der Nut (22) der Führungsbolzen (16 bis 19) etwa dem Radius der auf den Nuten laufenden Stäbe (5, 6) des Korbes (1) entspricht.

## Claims

1. Arrangement for the storage of containers or the like for sterile articles, with a guide device inserted in a cupboard, a shelf, or the like for guiding a basket (1), characterized in that the guide device is composed of guide pins (16 to 19) which define a guide plane, in that the basket (1) has guide slots (10,11) extending parallel to the guide plane, in that the guide slots (10, 11) are formed in the region of the corners of the basket (1) at least diagonally relative to one another, and in that the spacing of the guide pins (16, to 19) in the direction of movement of the basket (1) is smaller than the length (a) of the basket (1) by approximately the length of the guide slots (10, 11).

2. Arrangement according to Claim 1, characterized in that each guide slot (10, 11) is open towards the centre of the basket (1).

3. Arrangement according to Claim 1 or Claim 2, characterized in that each guide slot (10, 11) is defined by a hoop (12, 14) or the like extending along the side of the basket (1).

4. Arrangement according to at least one of the preceding claims, characterized in that the guide pins (16 to 19) are provided with a guide groove (22).

5. Arrangement according to Claim 4, characterized in that each guide pin (16 to 19) has a respective circumferential guide groove (22).

6. Arrangement according to at least one of the preceding claims, characterized in that the guide pins (16 to 19) are welded to a frame or the like.

7. Arrangement according to Claim 3, characterized in that the hoops (12, 14) are provided at the sides of the basket (1).

8. Arrangement according to at least one of the preceding claims, characterized in that the curvature of the groove (22) of the guide pins (16, to 19) corresponds approximately to the radius of the rods (5, 6) of the basket (1) which run on the grooves.

## Revendications

1. Dispositif de stockage pour des récipients stériles ou similaires, comprenant un système de guidage, monté dans une armoire, un rayonnage ou un dispositif similaire, destiné à guider un panier (1)
caractérisé en ce que le système de guidage est formé de goupilles de guidage (16 à 19), qui définissent un plan de guidage, en ce que le panier (1) est muni de rainures de guidage (10, 11) parallèles au plan de guidage, en ce que les rainures de guidage (10, 11) s'étendent au moins diagonalement l'une par rapport à l'autre dans la zone des angles du panier (1), en ce que les goupilles de guidage (16 à 19) sont situées, dans le sens du déplacement du panier (1), à une certaine distance les unes des autres, laquelle est inférieure à la longueur du panier (1) d'une valeur égale à la longueur de la rainure de guidage (10, 11) environ.

2. Dispositif selon la revendication 1, caractérisé en ce que chaque rainure de guidage (10, 11) est ouverte en direction du milieu du panier (1).

3. Dispositif selon la revendication 1 ou 2, caractérisé en ce que chaque rainure de guidage (10, 11) est fixée par un étrier (12, 14) ou un élément similaire qui s'étend sur les parties latérales du panier (1).

4. Dispositif selon l'une quelconque des revendications précédentes, caractérisé en ce que les goupilles de guidage (16 à 19) sont munies d'une rainure de guidage (22).

5. Dispositif selon la revendication 4, caractérisé en ce que les goupilles de guidage (16 à 19) sont munies chacune d'une rainure de guidage périphérique (22).

6. Dispositif selon l'une quelconque des revendications précédentes, caractérisé en ce que les goupilles de guidage (16 à 19) sont soudées à un cadre ou un élément similaire.

7. Dispositif selon la revendication 3, caractérisé en ce qu'il est prévu de monter les étriers (12, 14) sur les parties latérales du panier (1).

8. Dispositif selon l'une quelconque des revendications précédentes, caractérisé en ce que la courbure de la rainure (22) des goupilles de guidage (16 à 19) correspond pratiquement au rayon des tiges (5, 6) du panier (1), qui se déplacent dans les rainures.
